Europäisches Patentamt

European Patent Office

Office européen des brevets

(19)

(11) Veröffentlichungsnummer: **0 071 976**
A1

# (12) EUROPÄISCHE PATENTANMELDUNG

(21) Anmeldenummer: 82107048.9

(22) Anmeldetag: 04.08.82

(51) Int. Cl.³: **G 01 N 33/58**, G 01 N 33/54

(30) Priorität: 05.08.81 CH 5048/81
02.11.81 CH 6989/81

(43) Veröffentlichungstag der Anmeldung: 16.02.83
Patentblatt 83/7

(84) Benannte Vertragsstaaten: **AT BE CH DE FR GB IT LI NL SE**

(71) Anmelder: **F. HOFFMANN-LA ROCHE & CO. Aktiengesellschaft, CH-4002 Basel (CH)**

(72) Erfinder: **Burckhardt, Johann Jakob, Dr., Im Zelg 6, CH-4144 Arlesheim (CH)**
Erfinder: **Staehelin, Theophil, Prof.Dr., Hangstrasse 34, CH-4144 Arlesheim (CH)**
Erfinder: **Stocker, John, Dr., Höhenweg 9, CH-4113 Flüh (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

(54) **Markiertes, immunologisch aktives Mittel, dessen Herstellung und dessen Verwendung in einem immunologischen Nachweis- oder Bestimmungsverfahren.**

(57) Es wird ein markiertes, immunologisch aktives Mittel, ein Verfahren zur Herstellung dieses Mittels, ein immunologisches Verfahren zum Nachweisen bzw. Bestimmen einer Substanz mit diesem Mittel sowie die Verwendung dieses Mittels in einem immunologischen Nachweisverfahren beschrieben. Das erwähnte Mittel ist ein Komplex bestehend aus einem immunologisch aktiven Material, an das Biotin kovalent gebunden ist, und Avidin, an das ein Enzym kovalent gebunden ist.

EP 0 071 976 A1

0071976

4. Aug. 1982

RAN 4093/62

F.HOFFMANN-LA ROCHE & CO. Aktiengesellschaft, Basel (Schweiz)

## Markiertes, immunologisch aktives Mittel, dessen Herstellung und dessen Verwendung in einem immuno- logischen Nachweis- oder Bestimmungsverfahren

Die vorliegende Erfindung betrifft ein markiertes, immunologisch aktives Mittel, ein Verfahren zur Herstellung dieses Mittels, ein immunologisches Verfahren zum Nachweisen bzw. Bestimmen einer Substanz mit diesem Mittel sowie die Verwendung dieses Mittels in einem immunologischen Nachweisverfahren.

Es ist bekannt, dass sich viele immunologisch aktive Stoffe, wie z.B. Antikörper, nicht ohne Verlust deren immunologischer Aktivität mit z.B. $^{125}J$ oder mit Peroxidase markieren lassen. Dies trifft insbesondere für die nach der bekannten Methode von Köhler und Milstein (Nature, 256, 495f, 1975) erhaltenen monoklonalen Antikörper zu.

Es besteht demgemäss ein Bedürfnis, eine universelle Methode zu finden, nach der immunologisch aktive Materialien ohne Verlust ihrer immunologischen Aktivität markiert werden können.

Klt/ 1.6.82

0071976

Im Rahmen der vorliegenden Erfindung wurde nun gefunden, dass man Biotin kovalent an immunologisch aktive Materialien binden kann, und dass man daran Avidin, an das ein Enzym kovalent gebunden ist, binden kann.

Die vorliegende Erfindung betrifft demnach einen Komplex, der aus einem immunologisch aktiven Material, an das Biotin kovalent gebunden ist, und Avidin, an das ein Enzym kovalent gebunden ist, besteht.

Als immunologisch aktive Materialien sind insbesondere Antikörper oder Antigene geeignet. Besonders geeignet sind monoklonale Mausantikörper. Bei den monoklonalen Mausantikörpern haben sich insbesondere die als bevorzugt erwiesen, die gegen das Hepatitis B Virus Oberflächenantigen gerichtet sind, oder diejenigen, die gegen das carcinoembryonale Antigen (CEA) gerichtet sind.

Im Rahmen der vorliegenden Erfindung kommen alle Enzyme in Betracht, die bei immunologischen Methoden verwendet werden, d.h. also z.B. alkalische Phosphatase, Malat-Dehydrogenase, Glukose-6-phosphat-dehydrogenase, Glukose-oxidase, Glukoamylase, Galactosidase, Acetylcholinesterase sowie Peroxidase. Als besonders bevorzugt hat sich hierbei die Peroxidase erwiesen. Speziell geeignet ist die Peroxidase aus Meerrettich.

Das Verfahren zur Herstellung des erfindungsgemässen Komplexes besteht darin, dass man Biotin kovalent an ein immunologisch aktives Material bindet, Avidin kovalent an ein Enzym bindet und die beiden erhaltenen Produkte miteinander mischt. Der erhaltene Komplex kann anschliessend in an sich bekannter Weise lyophilisiert werden.

Die kovalente Bindung von Biotin an das immunologisch aktive Material kann in an sich bekannter Weise erfolgen, indem man das immunologisch aktive Material in schwach basischer, gepufferter Lösung mit einer Lösung von Biotin-Succinimidester in Dimethylsulfoxid versetzt. Zur Reinigung des
Produktes kann man anschliessend gegen phosphatgepufferte
Kochsalzlösung dialysieren. Die phosphatgepufferte Kochsalzlösung kann Desinfektionsmittel, wie "Thimerosal" (Fluka)
oder Merfen enthalten.

Da das Verhältnis zwischen dem immunologisch aktiven
Material und dem Biotin-Succinimidester für die erfolgreiche Umsetzung kritisch ist, muss dieses Verhältnis für
jedes immunologisch aktive Material besonders bestimmt werden.

Die Kopplung des Enzyms, insbesondere der Meerrettich-
Peroxidase, an das Avidin kann nach bekannten Methoden, z.B.
nach der Methode von Wilson und Nakane in "Immunfluorescence
and Related Staining Techniques", 1978, Seite 215 erfolgen.

Zur Herstellung des erfindungsgemässen Komplexes kann
eine Lösung des immunologisch aktiven Materials, an das
Biotin kovalent gebunden ist, mit einer Lösung von Avidin,
an das das Enzym kovalent gebunden ist, versetzt werden. Die
Umsetzung kann bei Raumtemperatur erfolgen. Zur Stabilisierung des
gebildeten Komplexes kann dieser in an sich bekannter
Weise lyophilisiert werden.

Der erfindungsgemässe Komplex kann in jedem immunologischen
Nachweisverfahren verwendet werden. Als besonders geeignetes
Verfahren hat sich das sogenannte Sandwichverfahren erwiesen.

Die Erfindung betrifft demnach weiterhin ein Sandwichverfahren zum Nachweisen bzw. Bestimmen einer Substanz,
welches dadurch gekennzeichnet ist, dass die zu bestimmende
Substanz mit einem immunologisch aktiven Reaktionspartner,
der an einen wasserunlöslichen Träger gebunden ist oder wird,
und einem Komplex bestehend aus einem immunologisch aktiven
Material, an das Biotin gebunden ist, und Avidin, an das ein
Enzym kovalent gebunden ist, inkubiert wird, und dass nach
Trennen von fester und flüssiger Phase entweder in der festen oder in der flüssigen Phase die enzymatische Aktivität gemessen wird, und daraus auf das Vorhandensein bzw. das Ausmass der zu bestimmenden Substanz geschlossen wird.

Bei diesem Sandwichverfahren kommen als zu bestimmende Substanzen insbesondere Antigene in Betracht. Als besonders bevorzugte Antigene haben sich das Hepatitis B Virus Oberflächenantigen sowie das carcinoembryonale Antigen (CEA) erwiesen.

Bei diesem Sandwichverfahren werden bevorzugt als immunologisch aktive Reaktionspartner zwei verschiedene Antikörper verwendet, die gegen das gleiche Antigen, aber verschiedene Epitope dieses Antigens gerichtet sind. Hierzu kommen insbesondere zwei verschiedene monoklonale Mausantikörper in Betracht.

Die nachfolgenden Beispiele erläutern die Erfindung.

Die in den Beispielen verwendeten monoklonalen anti-Hepatitis B Virus Oberflächenantigen Antikörper (anti-HBs) erhält man nach der Methode von Fazekas et al. in Journal of Immunological Methods, 35, 1-21 (1980), wobei die Mäuse mit Hepatitis B Virus Oberflächenantigen immunisiert werden. Zur Fusion wird die von Fazekas (loc. cit.) beschriebene Myelomlinie FO verwendet. Die erhaltenen Hybridome werden in an sich bekannter Weise i.p. in BALB/c Mäuse gespritzt, und die gebildete Aszites-Flüssigkeit wird zur Gewinnung der Antikörper verwendet. Für die Beispiele werden zwei Antikörper verwendet, die gegen verschiedene Epitope von Hepatitis B Virus Oberflächenantigen gerichtet sind, und die als anti-HBs-1 bzw. anti-HBs-2 bezeichnet werden. Die IgG-Fraktion der Antikörper wird durch Ammoniumsulfatfällung und Chromatographie an Diäthylaminoäthylzellulose von Aszites-Flüssigkeit gewonnen.

Die in den Beispielen verwendeten monoklonalen Maus Anti-CEA Antikörper erhält man ebenfalls in Analogie zu der In Journal of Immunological Methods, 32 (1980) 297-304 beschriebenen Methode, wobei jedoch als Ausgangszelllinie für die Fusion die Myelomlinie Sp 2/01-Ag verwendet wird, welche unter der Nr CRL 8006 bei ATCC am 25.5.1979 hinterlegt und am 24.11.81 der Oeffentlichkeit uneingeschränkt zugänglich gemacht worden ist. Die Fusion erfolgt mit Milzzellen von Mäusen, die mit CEA immunisiert wurden. Die Immunisierung der Mäuse erfolgte in Analogie zu Tabelle 1 der erwähnten Publikation, wobei die ersten beiden Immunisierungen mit je 50 µg CEA erfolgten, die Immunisierungen 3 und 4 weggelassen wurden, Immunisierung 5 mit 50 µg CEA und die Immunisierungen 6-8 mit je 200 µg CEA erfolgen. Es werden zwei verschiedene, geeignete monoklonale Antikörper verwendet, die gegen unterschiedliche Epitope des CEA Antigens gerichtet sind und die als anti-CEA(C-3) bzw. anti-CEA(C-19) bezeichnet werden.

## Beispiel 1

Die IgG-Fraktion von anti-HBs-1 bzw. anti-CEA (C-3)
Antikörpern wird über Nacht bei 2-8°C gegen 0.1 mol/1
Natriumbikarbonatlösung (pH 8.2-8.5) dialysiert und auf eine
Proteinkonzentration von 1 mg/ml eingestellt. Dazu werden
pro ml Proteinlösung 120 µl einer ganz frisch hergestellten
Biotin-Succinimidester-Lösung (1 mg/ml in Dimethylsulfoxid)
zugemischt und während 4 Stunden bei Zimmertemperatur
inkubiert. Anschliessend wird das Biotin-Antikörper-
Konjugat bei 2-8°C gegen phosphatgepufferte, physiologische
Kochsalzlösung mit 0,66 mg/1 Merfen dialysiert. Als Proteinstabilisator werden 10 mg/ml Rinderserumalbumin beigegeben.

Für die Kopplung von Peroxidase an Avidin werden 5 mg
Avidin in 2.5 ml einer 0.1 mol/1 Natriumbikarbonatlösung
(pH 9.5) gelöst und über Nacht bei 2-8°C gegen die gleiche
Lösung dialysiert. 10 mg Peroxidase aus Meerrettich werden
in 3.0 ml dest. Wasser gelöst, während einer Stunde bei
Raumtemperatur stehen gelassen, dann mit 0.5 ml einer
wässrigen, frisch hergestellten 0.1 mol/1 Natriumperjodatlösung vermischt und genau 20 Minuten bei Raumtemperatur
inkubiert. Sodann wird die Peroxidase durch Ultrafiltration
vom überschüssigen Natriumperjodat befreit und auf das
ursprüngliche Volumen konzentriert. Nach der Ultrafiltration
wird die Peroxidaselösung der Avidinlösung zugemischt,
worauf man während 2 Stunden bei Raumtemperatur inkubiert.
Zur Reduktion der Schiff'schen Basen und damit zur Stabilisierung der kovalenten Bindung werden der Avidin-Peroxidaselösung 0.5 ml einer frisch hergestellten, wässrigen,
0.1 mol/1 Natriumborhydridlösung zugemischt, worauf man
während mindestens 2 Stunden bei 2-8°C inkubiert. Anschliessend
wird das Avidin-Peroxidase-Konjugat gegen phosphatgepufferte,
physiologische Kochsalzlösung mit 0.66 mg/1 Merfen
dialysiert und mit 10 mg/ml Rinderserumalbumin stabilisiert.

0071976

Zur Bildung des Komplexes werden 55 μl anti-HBs-1-Antikörper-Biotin-Konjugat bzw. 55 μl anti-CEA (C-3) Antikörper-Biotin-Konjugat mit 48 μl Avidin-Peroxidase-Konjugat gemischt und 15 Minuten bei Raumtemperatur inkubiert. Die erfolgte Bildung des Komplexes kann kontrolliert werden, indem ein Teil der Lösung - nach geeigneter Verdünnung mit phosphatgepufferter, physiologischer Kochsalzlösung - mit anti-Mäuse IgG Antikörpern, die an einen unlöslichen Träger gebunden sind, inkubiert wird. Ist die Bildung der Komplexe erfolgt, so kann, nach Abtrennung der Flüssigphase, die Enzymaktivität in bekannter Weise an der Festphase nachgewiesen werden.

Der Komplex kann in herkömmlicher Weise lyophilisiert werden.

Für die erfindungsgemässe Verwendung wird der Komplex im Falle des anti-HBs-1-Antikörpers mit einer Lösung, die 0,25 mol/l Natriumdihydrogenphosphat, 10 g/l Rinderserumalbumin und 0,66 mg/l Merfen und 0,04 g/l 4-Amino-antipyrin enthält, verdünnt und auf pH 7,0 eingestellt und im Falle des anti-CEA (C-3) Antikörpers mit einer Lösung, die 0,20 mol/l Natriumdihydrogenphosphat, 2 g/l Rinderserumalbumin, 20% Ziegenserum, 0,5 g/l "Thimerosal" und 0,1 g/l 4-Amino-antipyrin enthält, verdünnt und auf pH 6,5 eingestellt.

0071976

## Beispiel 2

Zum Nachweis von Hepatitis B Virus Oberflächenantigen in menschlichen Seren oder Plasmen werden 0.05-0.15 ml der Probe in einer Rundbodenmikrotiterplatte mit Polystyrolkugeln von 3.2 mm Durchmesser, die mit der IgG-Fraktion von anti-HBs-2-Antikörpern beschichtet sind, und 0.05 ml des in Beispiel 1 beschriebenen Komplexes während einer Stunde bei 45°C inkubiert. Der Komplex wird mit der in Beispiel 1 beschriebenen Verdünnungslösung 1/500 verdünnt und in dieser Verdünnung eingesetzt. Nach der Inkubation werden die Polystyrolkugeln in der Titerplatte mit dest. Wasser gewaschen, worauf die Peroxidaseaktivität an der Festphase durch Inkubation der Polystyrolkugeln in 0.1 ml o-Phenylendiamin (4 mg/ml, in 0.1 mol/l Kaliumcitratpuffer, pH 5.0, mit 6 mmol/l Wasserstoffperoxid) ermittelt wird. Die Enzymreaktion wird nach 30 Minuten durch Zugeben von 0.1 ml 4 N Schwefelsäure und 2.5 mol/l Kochsalz gestoppt, und die Farbreaktion wird von Auge oder mit einem für Mikrotiterplatten geeigneten Photometer abgelesen. Durch Vergleich mit positiven oder negativen Proben wird ermittelt, ob die zu untersuchende Probe Hepatitis B Virus Oberflächenantigen aufweist.

## Beispiel 3

Zum Nachweis von CEA in menschlichen Seren oder Plasmen werden in die erforderliche Anzahl Teströhrchen (10x75 mm) je 0,2 ml Testlösung (0,2 mol/l Natriumphosphatpuffer vom pH 6,5 mit 2 g/l Rinderserumalbumin, 20% normales Ziegenserum, 0,1 g/l 4-Amino-antipyrin und 0,5 g/l "Thimerosal" und 480 ng/ml monoklonales Maus Anti-CEA-(C-3)-Biotin/Avidin-Peroxidase) pipettiert, 0,050 ml der zu analysierenden Patientenproben bzw. der CEA-Standards (0 ng/ml CEA, 2,5 ng/ml CEA, 5,0 ng/ml CEA, 10 ng/ml CEA und 20 ng/ml CEA) und des CEA-Kontrollserums (5,0 ng/ml CEA) zugemischt, je eine mit monoklonalem Maus Anti-CEA-(C-19)-sensibilisierte Polystyrolkugel (Ø = 6,5 mm) zugefügt und bei 37°C während 16 Stunden inkubiert. Anschliessend werden die Polystyrolkugeln dreimal mit je 2-5 ml dest. Wasser gewaschen, in je 0,5 ml Substratpuffer (0,1 mol/l Kaliumcitrat vom pH 5,0 mit 6 mmol/l $H_2O_2$ und 40 mmol/l o-Phenylendiamin) für die Aktivitätsbestimmung der an die Kugel immunologisch gebundenen Peroxidase transferiert und während 30 Minuten bei 18-26°C inkubiert. Zum Abstoppen der peroxidatischen Aktivität sowie zur Steigerung der Farbintensität werden 2,0 ml 1 N HCl zugemischt und innerhalb von 30 Minuten die Extinktion bei der Wellenlänge 492 nm photometrisch gemessen. In der Tabelle 1 sind die Werte einer CEA-Bestimmung aufgeführt und mit den Werten verglichen, wie sie mit dem Radioimmunoassay von ROCHE erhalten wurden.

0071976

Tabelle 1

| Probematerial | $\Delta E_{492}$ nm/20$^o$C/30 Min. | |
|---|---|---|
| CEA-Standard | | |
| 0 ng/ml CEA | 0,080 / 0,080 | |
| 2.5 " " | 0,180 / 0,185 | |
| 5.0 " " | 0,230 / 0,235 | |
| 10 " " | 0,430 / 0,450 | |
| 20 " " | 0,850 / 0,820 | |
| CEA-Kontrollserum | | |
| 5 ng/ml CEA | 0,240 / 0,235 | |
| Patientenplasmen | ROCHE-RIA-Test | erfindungsgemässe Verfahren |
| 3157 | 3,2 ng/ml | 2,8 ng/ml |
| 3368 | 7,0 " | 7,2 " |
| 3395 | 14,7 " | 15,0 " |
| 3401 | 8,6 " | 8,9 " |
| 3410 | 22,5 " | 20,5 " |
| 3419 | 4,2 " | 4,5 " |
| 3679 | 5,7 " | 5,5 " |

0071976

## Patentansprüche

1. Komplex bestehend aus einem immunologisch aktiven Material, an das Biotin kovalent gebunden ist, und Avidin, an das ein Enzym kovalent gebunden ist.

2. Komplex nach Anspruch 1, dadurch gekennzeichnet, dass das immunologisch aktive Material ein Antikörper oder ein Antigen ist.

3. Komplex nach Anspruch 2, dadurch gekennzeichnet, dass der Antikörper ein monoklonaler Maus-Antikörper ist.

4. Komplex nach Anspruch 3, dadurch gekennzeichnet, dass der monoklonale Maus-Antikörper ein Antikörper gegen das Hepatitis B Virus Oberflächenantigen ist.

5. Komplex nach einem der Ansprüche 1 bis 4, dadurch gekennzeichnet, dass das Enzym Peroxidase ist.

6. Komplex nach einem der Ansprüche 1 bis 5, dadurch gekennzeichnet, dass die Peroxidase Meerrettich-Peroxidase ist.

7. Komplex nach einem der Ansprüche 1 bis 6, dadurch gekennzeichnet, dass der Komplex in lyophilisierter Form vorliegt.

8. Verfahren zur Herstellung eines Komplexes nach Anspruch 1, dadurch gekennzeichnet, dass man Biotin kovalent an ein immunologisch aktives Material bindet, Avidin kovalent an ein Enzym bindet und die beiden erhaltenen Produkte miteinander mischt.

9. Verfahren nach Anspruch 8, dadurch gekennzeichnet, dass man den erhaltenen Komplex lyophilisiert.

0071976

10. Sandwichverfahren zum Nachweisen bzw. Bestimmen einer Substanz, dadurch gekennzeichnet, dass die zu bestimmende Substanz mit einem immunologisch aktiven Reaktionspartner, der an einen wasserunlöslichen Träger gebunden ist oder wird, und einem Komplex bestehend aus einem immunologisch aktiven Material, an das Biotin kovalent gebunden ist, und Avidin, an das ein Enzym kovalent gebunden ist, inkubiert wird und dass nach Trennen von fester und flüssiger Phase entweder in der festen oder in der flüssigen Phase die enzymatische Aktivität gemessen wird, und daraus auf das Vorhandensein bzw. das Ausmass der zu bestimmenden Substanz geschlossen wird.

11. Verfahren nach Anspruch 10, dadurch gekennzeichnet, dass die zu bestimmende Substanz ein Antigen ist.

12. Verfahren nach Anspruch 11, dadurch gekennzeichnet, dass das Antigen Hepatitis B Virus Oberflächenantigen ist.

13. Verfahren nach einem der Ansprüche 10 bis 12, dadurch gekennzeichnet, dass die immunologisch aktiven Reaktionspartner verschiedene Antikörper sind, die gegen das gleiche Antigen aber verschiedene Epitope dieses Antigens gerichtet sind.

14. Verfahren nach Anspruch 13, dadurch gekennzeichnet, dass die Antikörper zwei verschiedene monoklonale Maus-Antikörper sind.

15. Verfahren nach Anspruch 14, dadurch gekennzeichnet, dass die monoklonalen Maus-Antikörper anti-Hepatitis B Virus Oberflächenantigen Antikörper sind.

16. Verfahren nach einem der Ansprüche 10 bis 15, dadurch gekennzeichnet, dass das Enzym Peroxidase ist.

17. Verfahren nach Anspruch 16, dadurch gekennzeichnet, dass die Peroxidase Meerrettich-Peroxidase ist.

**Europäisches Patentamt**

**EUROPÄISCHER RECHERCHENBERICHT**

007.1976 Nummer der Anmeldung

EP 82 10 7048

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | --- | | G 01 N 33/58 |
| X | US-A-4 228 237 (R.C. HEVEY et al.)<br><br>* Zusammenfassung; Spalte 1, Zeile 34 - Spalte 5, Zeile 5; Spalte 5, Zeilen 23-40, 49-58, 65-68; Spalte 6, Zeilen 1-19; Beispiele II-IV,VII-VIII; Ansprüche 1-10, 16-19, 21-28 * | 1-6,8-11,16, 17 | G 01 N 33/54 |
| | --- | | |
| X | THE JOURNAL OF HISTOCHEMISTRY AND CYTOCHEMISTRY, Band 27, Nr. 8, August 1979, Seiten 1131-1139, The Histochemical Society, Inc., USA J.-L. GUESDON et al.: "The use of avidin-biotin interaction in immunoenzymatic techniques" * Insgesamt * | 1-5,8, 10,11, 16 | |
| | | | RECHERCHIERTE SACHGEBIETE (Int. Cl. ³) |
| | --- | | |
| A | CHEMICAL ABSTRACTS, Band 94, Nr. 23, 8. Juni 1981, Seite 469, Nr. 190054g, Columbus, Ohio, USA M. UOTILA et al.: "Two-site sandwich enzyme immunoassay with monoclonal antibodies to human alpha-fetoprotein" & J. IMMUNOL. METHODS 1981, 42(1), 11-15 * Zusammenfassung * | 10,11, 13-15 | G 01 N |
| | --- -/- | | |

Der vorliegende Recherchenbericht wurde für alle Patentansprüche erstellt.

| Recherchenort | Abschlußdatum der Recherche | Prüfer |
|---|---|---|
| DEN HAAG | 09-11-1982 | GRIFFITH G. |

Europäisches
Patentamt

**EUROPÄISCHER RECHERCHENBERICHT**

0071976

Nummer der Anmeldung

EP 82 10 7048

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | GASTROENTEROLOGY, Band 79, Nr. 5, Teil 2, November 1980, Seite 1063, J.R. WANDS et al.: "Identification of epitopes on HBsAg polypeptides by analysis with monoclonal anti-HBs antibodies" * Insgesamt * | 12-15 | |
| | --- | | |
| X | CHEMICAL ABSTRACTS, Band 94, Nr. 21, 25. Mai 1981, Seite 288, Nr. 169970t, Columbus, Ohio, USA K. YASUDA et al.: "Use of peroxidase-avidin conjugate for the demonstration of intracellular antigen" & EXPERIENTIA 1981, 37(3), 306-308 * Zusammenfassung * | 1,2,5, 8 | |
| | --- | | RECHERCHIERTE SACHGEBIETE (Int. Cl. ³) |
| A | EP-A-0 005 271 (THE WELLCOME FOUNDATION LTD.) * Zusammenfassung; Seite 3, Zeile 18 - Seite 4, Zeile 21 * | 1,10 | |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 09-11-1982 | GRIFFITH G. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsatze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein- stimmendes Dokument

EPA Form 1503. 03 82